# EUROPEAN PATENT APPLICATION

(11) **EP 1 559 712 A1**
(43) Date of publication of application: **03.08.2005**
(21) Application number: 04300052.0
(22) Date of filing: 28.01.2004
(51) Int. Cl.: C07D 311/16, G01N 33/68, C07K 7/08, C12P 21/02

(54) **A method for manufacturing optically active coumaryl amino acid salts and the coumaryl aminoacid salts thus obtained**

(71) Applicant: INSTITUT NATIONAL DE LA SANTE ET DE LA RECHERCHE MEDICALE (INSERM), 75013 Paris (FR)
(72) Inventor: Garbay, Christiane, 75011 Paris (FR); Bischoff, Laurent, 75014 Paris (FR); Brun, Marie-Priscille, 75015 Paris (FR)
(74) Representative: Catherine, Alain

(57) **Abstract**

The present invention relates to the field of the synthesis of optically active amino acids, mainly for the purpose of manufacturing optically active polypeptides that are useful as labelled detection tools. The manufacturing method involves the preparation of optically active coumaryl amino acid salts of formula (I).

## Description

### FIELD OF THE INVENTION

The present invention relates to the field of the synthesis of optically active amino acids, mainly for the purpose of manufacturing optically active polypeptides that are useful as labelled detection tools.

### BACKGROUND OF THE INVENTION

It is known in the art that fluorescent derivatives of biologically active peptides represent useful experimental tools for probing biological structure, function and interaction and for visualising intracellular processes or molecular interactions, specifically at the level of single cells. Fluorescence detection is inherently very selective because of the use of excitation and emission wavelengths which discriminate against fluorescence from non-proteic species. Fluorescence detection also offers the advantage of increased sensitivity, typically more than one order of magnitude, as compared to detection by UV spectroscopy. Moreover these peptide-based reporters combine fluorescence detection sensitivity with a high specificity as regards the identity of the polypeptides which are detected, since the choice of both the excitation and the emission wavelength specifically targets the fluorescently labelled amino acid which is sought.

Actually such fluorescently-labelled ligands have proven to be of value in the detection and localisation of receptors in tissues as well as for the studies of enzyme activities or interactions with binding domain.

For fluorescence detection to be used in peptide analysis, the peptide to be detected or monitored must either include a fluorescent natural amino acid, such as tryptophan or tyrosine, or else it must be labelled using extrinsic or intrinsic fluorescent probes. Labelling polypeptides by non-natural fluorophores characterised by specific excitation and/or emission wavelengths, which are distinct from those of the natural amino acids, avoids the concomitant fluorescence detection of Trp or Tyr.

Extrinsic fluorescent dyes are usually introduced into peptides by covalent coupling with the amino, carboxyl or thiol group present in their sequences.

Many commonly used fluorophores are known. For labelling the N-terminal amino group of polypeptides, fluorescein, rhodamine, NBD chloride (4-chloro-7-nitrobenzofuran), LYA (lucifer yellow anhydride or 4-amino-3,6-disulfo-1,8-naphtalic anhydride), dansyl derivatives or o-aminobenzoyl group are conventionally used. For labelling the C-terminal carboxy group, 3-(2,4-dinitrophenyl)-2,3-diaminopropionic amide group 7-amino-4-methyl coumarin or EDANS (5-((2-aminoethyl)amino)naphthalene-1- sulfonic acid, sodium salt) are conventionally used. For labelling polypeptides inside the amino acid chain, fluorophores may be incorporated on the side chain of cysteine or lysine residues, fluorescent groups such as, for example, BODIPY (boron dipyromethene difluoride) or SNAFL (carboxy-seminaphto-fluorescein) are used.

However, it is known that coupling fluorescent probes to the C- or N-terminal ends of a polypeptide often affects the spatial conformation and also the biological activities of said polypeptide. Thus, in order to avoid these deleterious alterations to the parent polypeptide, incorporation of the fluorescent dye in a well-chosen position into the target polypeptide is desirable. If the fluorescent core can be introduced into an amino acid, thus leading to an intrinsic probe, its usefulness would become considerable since it may be incorporated at any position into a biologically active peptide. It is also known that incorporating a fluorescent amino acid into a peptide offers at least two advantages : (i) first, such peptides can be synthesized by SPPS (Solid Phase Peptide Synthesis) and (ii) second, as both ends of these peptides are available for chemical reactions, other residues can be attached to one or both ends of the amino acid chain, notably for the purpose of increasing its solubility or its bio availability.

To date, several fluorescent amino acids have been described, that may be used for the synthesis of a fluorescent polypeptide through conventional chemical peptide synthesis. The most used fluorescent amino acid residue is the very hindered and hydrophobic 3-pyrenylalanine (Pya). Other fluorescent amino acid residues were also disclosed in the art, such as -anthraniloyl-L- , -diaminopropionic acid (atn Dap) and its derivatives, 3-[2-(phenyl)benzoxazol-5-yl]alanine derivatives (Box Ala), 4-ethoxymethylene-2-[1]naphtyl-5(4H)oxazolone derivatives and coumaryl amino acids such as (6,7-dimethoxy-4-coumaryl)alanine (Dmca) or (6-methoxy-4-coumaryl)alanine (Mca).

Coumarins have been extensively used to label amines, thiols and carboxyl groups. Although coumarin itself is not fluorescent, the addition of various electron donating groups ― particularly at the 6 and 7 positions ― results in a red shift and increased fluorescence intensity. For these reasons, the use of fluorescent coumarin derivatives is very widespread in biological and medical research, providing a useful tool in the search for new biologically-active compounds and in the development of new diagnosis methods. Coumarins are interesting fluorescent brightening agents and dyes owing to their good quantum yields, low bleaching, reasonable stability and relative ease of synthesis. The most commonly-encountered fluorescent coumarins either absorb in the UV region and emit blue light or are yellow dyes emitting a green fluorescence. Although several derivatives which both absorb and emit at longer wavelengths are known, there remains some interest in the molecular design and synthesis of new coumarin derivatives which would extend the available range of long-wavelength emitting fluorescent materials.

There have been known several routes towards the synthesis of the coumarin core itself, including Von Pechmann (1884), Perkin (1942) , Knoevenagel (1996), Reformatsky (1942) and Wittig (1998) reactions.

The Von Pechmann reaction is one of the most simple and straightforward methods for the preparation of coumarins *per se.* Classically, the method consists of the condensation of phenols with - ketoesters in the presence of a variety of Lewis or Brönsted acids. These include, for instance, sulfuric acid, aluminium chloride, trifluoroacetic acid and methanesulfonic acid. More recently, mild reaction conditions have been described, which use indium(III) chloride or ZnI₂ as a Lewis acid catalyst, these conditions being compatible with the presence of basic amino-groups.

In the art, there has also been disclosed enantioselective methods of synthesis of coumaryl amino acids through diastereoselective alkylation reactions of chiral glycine equivalents. Chiral auxiliary compounds disclosed by Oppolzer (1989) and Williams (1998) have been used for the synthesis of L-(6,7-dimethoxy-4-coumaryl)alanine Bennett et al., (1997) and L-(7-methoxy-4-coumaryl)alanine (Kele et al., 2000) respectively. These methods led to optically pure amino acids but involved numerous steps, including the prior synthesis of the alkylating agent. The shortest method of this kind known from the inventors allows the synthesis coumaryl- alanine or ethylglycine in two or three steps from suitably protected aspartic acid and glutamic acid, respectively. Such prior art methods of synthesis of substituted coumaryl alanine residues through an alkylation step is represented hereunder.

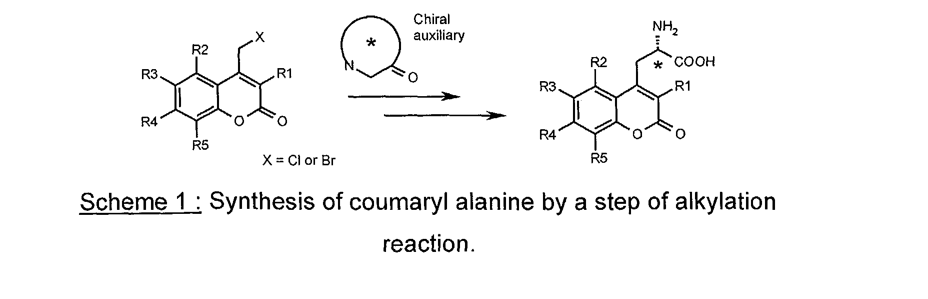

Although the prior art methods above of synthesis of coumaryl aminoacid residues are, globally, satisfactory, these prior art methods include numerous process steps, generally not less than 6 or 7 process steps, including the necessary steps of preparing the alkylation agent which is reacted with the coumarin derivative.

Further, the method step of diastereoselective alkylation of the coumarin derivative can only be performed by using a chirally pure auxilliary alkylating agent, such as disclosed in Scheme 1 above; the synthesis of which under a chirally pure form is uneasy and compulsorily includes steps of enantiomeric purification prior its use in the synthesis method.

There is thus a need in the art for more convenient, more specifically, and for more straightforward methods of synthesis of coumaryl amino acid derivatives. Such desirable methods should be simpler to perform and also be less expensive than those already known in the art.

### SUMMARY OF THE INVENTION

A first object of the invention consists of a method for manufacturing an optically active coumaryl amino acid salt having the following formula (I) : 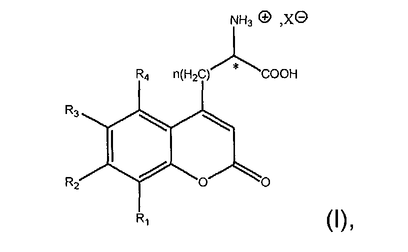 wherein :
(i) n is an integer ranging from 1 to 2 ;
(ii) X⁻ represents Cl⁻, Br⁻, I⁻, CH₃SO₃⁻, CF₃CO₂⁻, CF₃SO₃⁻ ;
(iii) R₁ represents H, halogen, alkyl, acyl, nitrile, sulfonate, aminosulfonyl, carbonyl and carbamoyl, OH, O- or N- substituted by alkyl or acyl group ;
(iv) R₂ represents H, halogen, alkyl, acyl, nitrile, sulfonate, aminosulfonyl, carbonyl and carbamoyl, OH, O- or N- substituted by alkyl or acyl group ;
(v) R₃ represents H, halogen, alkyl, acyl, nitrile, sulfonate, aminosulfonyl, carbonyl and carbamoyl, OH, O- or N- substituted by alkyl or acyl group ;
(vi) R₄ represents H, halogen, alkyl, acyl, nitrile, sulfonate, aminosulfonyl, carbonyl and carbamoyl, OH, O- or N- substituted by alkyl or acyl group ; and
(vii) "*" represents the position of an asymmetric carbon atom;
wherein said method comprises the step of:
(b) reacting the amino acid -ketoester of the following formula (1) : 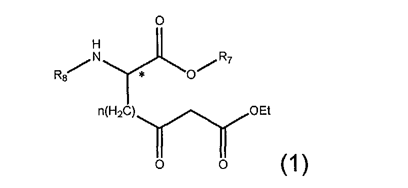 wherein R₇ and R₈ both mean, independently one from each other, a protective group,
with a substituted phenol of the following formula (11) : 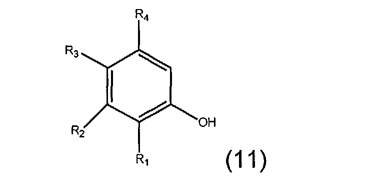 in the presence of methanesulfonic acid, for obtaining the compound of formula (I).

The present invention also relates to the novel compounds of formula (I) which are obtained by the method of synthesis above, as well as to the intermediate compounds which are useful for performing said method.

The invention also relates to protected coumaryl amino acid derivatives, which are obtained by using the compounds of formula (I) as starting material, and wherein said protected coumaryl amino acid derivatives are useful for the synthesis of the final optically active polypeptides.

The invention also relates to kits comprising a compound of formula (I), or a protected derivative thereof, as the starting material for the synthesis of an optically active polypeptide containing one or more coumaryl amino acid derivatives within their amino acid chain.

The invention also pertains to the optically active polypeptides synthesised using the coumaryl amino acid derivatives described therein as well as to detection kits containing those optically active polypeptides.

### BRIEF DESCRIPTION OF THE DRAWINGS

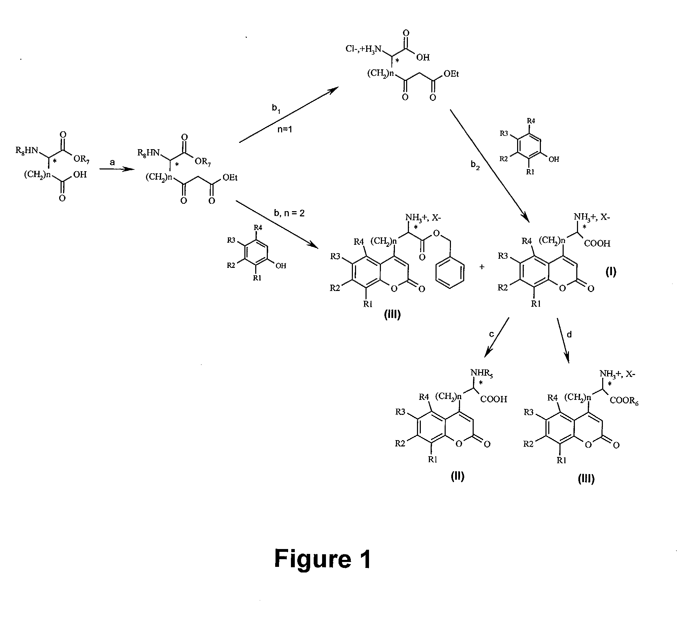

Figure 1 depicts the method of synthesis of the coumaryl amino acid derivatives according to the invention.

Step (a) consists of the synthesis of the -ketoester amino acid derivative which is used as the starting material of the general method.

By performing step (b), there is a simultaneous deprotection of the ester moiety of the -ketoester amino acid prepared according to step (a) and a condensation reaction of the deprotected activated resulting compound with a substituted phenol compound, whereby the coumaryl amino acid of formula (I) is obtained.

For the synthesis of the coumaryl aspartic acid derivative consisting of a specific embodiment of the compound of formula (I), there may be also performed steps (b1) and (b2), successively.

Then, starting from the coumaryl amino acid derivative of formula (I), protected coumaryl amino acid derivatives may be obtained, respectively a derivative comprising a protected amino group through performing step (c) and a derivative comprising a protected carboxy group through performing step (d).

### DETAILED DESCRIPTION OF THE INVENTION

Surprisingly, it has been found according to the invention that coumaryl amino acid derivatives, and specifically coumaryl aspartic acid or coumaryl glutamic acid derivatives, could be obtained with a good yield and with a high purity, by using a chirally pure -ketoester amino acid derivative as the starting material and then by condensing said - ketoester amino acid derivative with a substituted phenol, through performing a one-step or a two-step method.

Further, It has been found surprisingly that optically pure chiral - ketoester amino acid derivatives are obtained in a one-step method when using, as the starting material, the corresponding chiral amino acids, specifically aspartic acid and glutamic acid, which are easily commercially available.

These findings have allowed the inventors to design a new method for the synthesis of chirally pure coumaryl amino acid derivatives, which is started with inexpensive commercially available chirally pure compounds, which method does not comprise any step intended to purify a mixture of compound isomers. This, notably, explains why the final coumaryl amino acid derivative is obtained with a high purity. Further, since said method comprises a limited number of synthesis steps, the final coumaryl amino acid derivative is obtained with a good yield. Yet further, the combined features of the method according to the invention has allowed the inventors to design a method of synthesis of coumaryl amino acid derivatives, which method can be easily performed, is rapid to perform and is inexpensive due to the availability, at a low cost, of the chirally pure starting amino acids.

Notably, according to such a new method of synthesis of coumaryl aspartic acid or glutamic acid derivatives, there is no need of prior low yield multi-step and expensive synthesis of high cost chiral alkylating agent as in the prior art methods.

A first object of the invention consists of a method for manufacturing an optically active coumaryl amino acid salt having the following formula (I) : 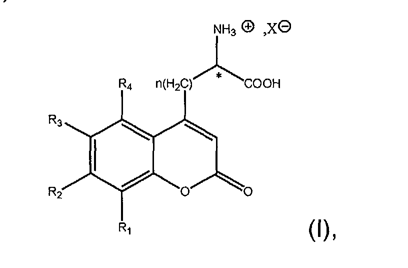 wherein :
(i) n is an integer ranging from 1 to 2 ;
(ii) X- represents Cl⁻, Br⁻, I⁻, CH₃SO₃⁻, CF₃CO₂⁻, CF₃SO₃⁻ ;
(iii) R₁ represents H, halogen, alkyl, acyl, nitrile, sulfonate, aminosulfonyl, carbonyl and carbamoyl, OH, O- or N- substituted by alkyl or acyl group ;
(iv) R₂ represents H, halogen, alkyl, acyl, nitrile, sulfonate, aminosulfonyl, carbonyl and carbamoyl, OH, O- or N- substituted by alkyl or acyl group ; (v) R₃ represents H, halogen, alkyl, acyl, nitrile, sulfonate, aminosulfonyl, carbonyl and carbamoyl, OH, O- or N- substituted by alkyl or acyl group ;
(vi) R₄ represents H, halogen, alkyl, acyl, nitrile, sulfonate, aminosulfonyl, carbonyl and carbamoyl, OH, O- or N- substituted by alkyl or acyl group ; and
(vii) "*" represents the position of an asymmetric carbon atom;
wherein said method comprises the step of :
(b) reacting the amino acid -ketoester of the following formula (1) : 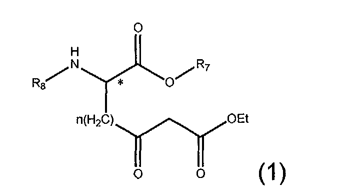 wherein R₇ and R₈ both mean, independently one from each other, a protective group,
with a substituted phenol of the following formula (11) : 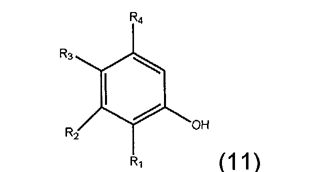 in the presence of methanesulfonic acid, for obtaining the compound of formula (I).

The fluorescent amino acids of formula (I) which are obtained according to the method of synthesis above are intended to prepare fluorescent polypeptides, through their incorporation within the polypeptide amino acid chain in place of a tyrosine residue. Compared with the natural tyrosine residue a fluorescent amino acid of formula (I) exhibits only a slight hindrance and possesses an emission wavelength higher than the emission wavelength of tyrosine and tryptophan residues, thus allowing the one skilled in the art to easily discriminate the fluorescent amino acids of the invention from the natural amino acids on the basis of their respective fluorescent properties.

As disclosed above, and in contrast to the prior art methods wherein the coumarin moiety is introduced by a chirally pure alkylating agent, the coumarin moiety is introduced according to the invention by condensation of a substituted phenol with the amino acid side chain of the corresponding starting amino acid, specifically the corresponding aspartic acid or glutamic acid residue.

According to the method of the invention, there occurs a transformation of the side-chain of Asp or Glu into a functional group suitable for cyclization with benzenic rings, specifically a Asp or Glu - ketoester suitable for cyclization with a substituted phenol, and then a condensation of the amino acid derived -ketoester thus obtained with one substituted phenol choosen among a large family of usable substituted phenols. Thus, the same -ketoester amino acid intermediate is used for the synthesis of numerous distinct coumaryl amino acid derivative compounds.

As a general embodiment of the method above, in step (d), protected amino acid derived -ketoesters obtained in step (b) were subjected to condensation with substituted phenols bearing R₁, R₂, R₃ and R₄ groups as defined above in formula (I) in the presence of methanesulfonic acid, leading to the synthesis of the chirally pure coumaryl amino acid derivative of formula (I).

Usually, at the end of step (b), the compound of formula (I) is found in a mixture with a second compound, wherein said second compound consists of a derivative of the compound of formula (I) but wherein the carboxy group is protected, said second compound having the formula (I-A) hereunder: 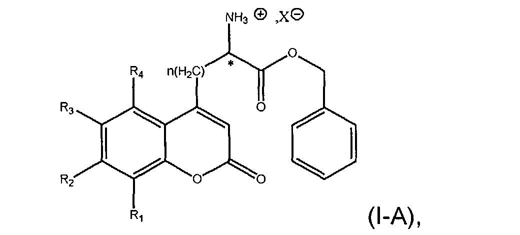 wherein R₁, R₂, R₃ and R₄ have the same meanings as for the compound of formula (I).

The mixture of compounds (I) and (III), which is obtained at the end of step (b), is mainly comprised of compound (I) . Typically, the ratio compound (I): compound (III), which is found at the end of step (b) of the method is 62:38.

Further, the content of compound (III) within the mixture of compounds cans be easily further lowered by performing step (b) with larger amounts of acid and substituted phenol, respectively.

Typically, at the end of step (b) of the method, a final mixture containing almost exclusively compound (III) can be obtained by using, as a large amount of acid, methanesulfonic acid at a concentration of at least 25 acid equivalents, and preferably at a concentration from 40 to 50 acid equivalents. By "acid equivalent", it is intended herein the number of equivalents, in moles, of methanesulfonic acid for carrying out the chemical reaction, which definition is well known from the one skilled in the art.

Still further, compound (I) is easily purified from the mixture of compounds (I) and (III), for example by performing a single step of purification on a chromatography column of silica gel, as it is disclosed in the examples.

In the above-described compounds (I) and (III), n, "*", X, R₁, R₂, R₃, R₄ have the same meaning as defined for the compound of formula (I).

According to the method of the invention, for the compound of formula (1), n is an integer ranging from 1 to 2.

As an alternative embodiment of step (b) of the method above, when the compound of formula (I) has the integer "n" meaning 1, said step may comprise two sub-steps that are successively performed, namely (i) in step (b1) the deprotection of the -ketoester amino acid derivative and then (ii) in step (b2) the condensation of the resulting activated product with the selected substituted phenol.

Thus, another object of the present invention consists of a method as generally defined above, wherein , for the compound of formula (1), n means 1 and step (d) comprises the following steps :
(b1) hydrogenolysis of the compound of formula (1) 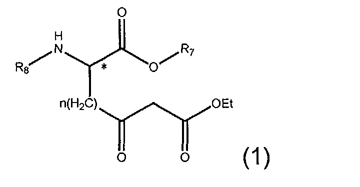 in the presence of catalytic palladium, for obtaining the compound of formula (2) 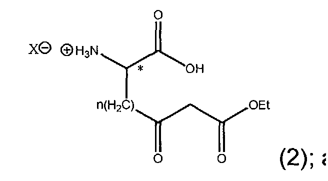 and
(b2) reacting the compound of formula (2) with the compound of formula (11 ) 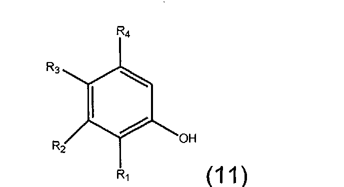 in the presence of methanesulfonic acid, for obtaining the compound of formula (I).

In step (b1), the protected amino acid derived -ketoester, which is used as the starting material, is subjected to hydrogenolysis over catalytic palladium on charcoal in order to achieve the deprotection of the amino and carboxyl functions, thus leading to deprotected amino acid Asp bearing a -ketoester moiety on its side chain, also named compound (2) throughout the present specification.

Accordingly, preferred examples of the compound (2) include (1 S)-1-carboxy-4-ethoxycarbonyl-3-oxo-butyl ammonium chloride and (1R)-1-carboxy-4-ethoxycarbonyl-3-oxo-butyl ammonium chloride.

In step (b2), the deprotected amino acid derived -ketoester obtained in step (d1) is subjected to condensation with a substituted phenol of formula (11) bearing R₁, R₂, R₃ and R₄ groups as defined above in formula (I), in the presence of methanesulfonic acid, leading to compound of formula (I).

In the just described compound (I), n is an integer ranging from 1 to 2 ; X- represents Cl⁻, Br⁻, I⁻, CH₃SO₃⁻, CF₃CO₂⁻, CF₃SO₃⁻ ; R₁ represents H, halogen, alkyl, acyl, nitrile, sulfonate, aminosulfonyl, carbonyl and carbamoyl, OH, O- or N- substituted by alkyl or acyl group ; R₂ represents H, halogen, alkyl, acyl, nitrile, sulfonate, aminosulfonyl, carbonyl and carbamoyl, OH, O- or N- substituted by alkyl or acyl group ; R₃ represents H, halogen, alkyl, acyl, nitrile, sulfonate, aminosulfonyl, carbonyl and carbamoyl, OH, O- or N- substituted by alkyl or acyl group ; R₄ represents H, halogen, alkyl, acyl, nitrile, sulfonate, aminosulfonyl, carbonyl and carbamoyl, OH, O- or N- substituted by alkyl or acyl group ; and represents the position of an asymmetric carbon atom.

Accordingly, examples of the compound (I) include (1S)-1-carboxy-2-(7-hydroxy-2-oxo-2H-chromen-4-yl)-ethyl ammonium trifluoroacetate (C1), (1S)-1-carboxy-2-(7-methoxy-2-oxo-2H-chromen-4-yl)-ethyl ammonium methanesulfonate (C2), (1R)-1-carboxy-2-(7-methoxy-2-oxo-2H-chromen-4-yl)-ethyl ammonium trifluoroacetate (C3), (1 S)-1-carboxy-2-(6-chloro, 7-hydroxy-2-oxo-2H-chromen-4-yl)-ethyl ammonium trifluoroacetate (C4), (1S)-1-carboxy-2-(7-ethoxy-2-oxo-2H-chromen-4-yl)-ethyl ammonium trifluoroacetate (C5), (1S)-1-carboxy-2-(5-hydroxy, 7-methoxy-2-oxo-2H-chromen-4-yl)-ethyl ammonium trifluoroacetate (C6), (1S)-1-carboxy-2-(7-hydroxy, 5-methoxy-2-oxo-2H-chromen-4-yl)-ethyl ammonium trifluoroacetate (C7), (1S)-1-carboxy-2-(5,7-dimethoxy-2-oxo-2H-chromen-4-yl)-ethyl ammonium trifluoroacetate (C8), (1S)-1-carboxy-2-(5,7-dihydroxy-2-oxo-2H-chromen-4-yl)-ethyl ammonium trifluoroacetate (C8), (1S)-1-ethoxycarbonyl-2-(6,7-dimethoxy-2-oxo-2H-chromen-4-yl)-ethyl ammonium trifluoroacetate (C9), (1S)-1-carboxy-2-(5-hydroxy 7-methoxy-2-oxo-2H-chromen-4-yl)-ethylammonium trifluoroacetate (C10), (1 S)-1-carboxy-3-(methoxy-2-oxo-2H-chromen-4-yl)-propyl ammonium trifluoroacetate (C11).

According to another chemical classification, which is more directly related to the amino acid nomenclature, compounds of formula (I) (1) to (8) above may also be termed as indicated below:
(C1) : L-(7-hydroxy-4-coumaryl)alanine, CF₃CO₂H,
(C2) : L-(7-methoxy-4-coumaryl)alanine, CH₃SO₃H,
(C3) : D-(7-methoxy-4-coumaryl)alanine, CF₃CO₂H,
(C4) : L-(6-chloro, 7-hydroxy-4-coumaryl)alanine, CF₃CO₂H,
(C5) : L-(7-ethoxy-4-coumaryl)alanine, CF₃CO₂H,
(C6) : L-(5-methoxy, 7-hydroxy-4-coumaryl)alanine, CF₃CO₂H,
(C7) : L-(5,7-dimethoxy-4-coumaryl)alanine, CF₃CO₂H,
(C8) : L-(5,7-dihydroxy-4-coumaryl)alanine, CF₃CO₂H.
(C9) : L-(6,7-dimethoxy-4-coumaryl)alanine, CF₃CO₂H;
(C10) : L-(5-hydroxy, 7-methoxy-4-coumaryl)alanine, CF₃CO₂H,
(C11) : L-(7-methoxy-4-coumaryl)ethylglycine, CF₃CO₂H.

In step (b2) , the condensation is carried out in the presence of an Brönsted acid i.e. any acid that acts as a proton donor in the chemical reaction. The acid is not particularly restricted. Examples thereof include concentrated hydrochloric acid, trifluoroacetic acid, triflic acid, methanesulfonic acid. Among all, best yields were and shortest reaction times were obtained with triflic or methanesulfonic acid. The amount of acid used is generally 25 equivalents.

Noticeably, in a preferred embodiment, the conditions wherein steps (b) and (b2) are performed are the same, excepted for the amount of acid which is used for performing step (b) which is 10 equivalents.

The reaction mixture can be diluted with a solvent. Examples thereof include dichloromethane (DCM), dimethylsulfoxide (DMSO) or dimethylether (DME) but it is preferably to carry the reaction with the acid as solvent.

The reaction temperature is generally room temperature, excepted for the addition of methanesulfonic acid which is completed at 0°C.

The reaction can be completed within one to five hours depending on the reactivity of the substituted phenol and the amount of acid used.

To achieve the highest yield synthesis of compound (I), the reaction should be carried out under restricted reaction conditions. The following method is particularly adequate therefore.

Method : In this method, amino acid derived -ketoesters and resorcinol (1.5 equiv) were mixed before addition, at 0°C, of 99% methanesulfonic acid (25 equiv) and stirred at room temperature for one to five hours. The deep-red, homogenous reaction mixture was taken up in cold ether (-30°C) and centrifuged 20 minutes at 3600 g. After ether discarding, the residue was washed and centrifuged again, the solid was taken up in water and lyophilised. The compounds obtained by this method were generally pure enough, otherwise they can be purified by semi-preparative HPLC, for example by using a gradient H₂O + TFA (0.1%)/H₂O: ACN (30:70) + TFA (0.09%). In this case, the trifluoroacetate salts were obtained instead of the methanesulfonate.

Most preferably, the -ketoester amino acid derivative which is used as the starting product in step (b) of the method is prepared by branching the -ketoester moiety on the side chain of a chirally pure corresponding amino acid having its carboxy- and amino- groups protected, specifically the protected chirally pure Asp or Glu residue, using the method of Brooks et al. (1979). Then, the resulting -ketoester amino acid derivative is deprotected on its carboxy- and amino- groups, for obtaining the desired compound (1).

Thus, according to a preferred embodiment of the method of the invention, wherein the amino acid -ketoester compound of formula (1) is obtained through the steps of :
(a1) subjecting a protected amino acid of the following formula (IV) 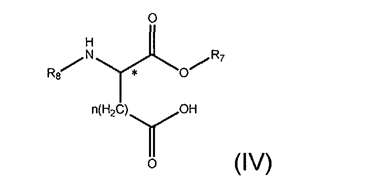 by treatment with carbonyldiimidazole; and
(a2) reacting the activated compound obtained at step (a1) with a salt of monoethyl malonic acid, for obtaining wherein the amino acid -ketoester compound of formula (1)

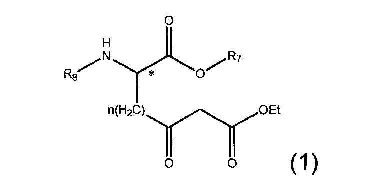

As already mentioned herein, it has unexpectedly be found according to the invention that performing the reaction of Brooks et al. (1979), starting from a chirally pure amino acid, specifically a chirally pure Asp or Glu, the product resulting from the reaction with the - ketoester moiety consists of -ketoester amino acid derivative of formula (1), which is also chirally pure. Indeed, the one skilled in the art should have expected that the resulting product of formula (1) would have consisted in a mixture of the two product isomers, which would have not been suitable for the final purpose of incorporating the final products of the method within the amino acid chain of a polypeptide, which amino acid chain must contain exclusively chirally pure amino acid residues.

In steps (a1) and (a2), amino acid-derived -ketoesters are synthesized, according to the efficient method described by Brooks and al. (1979). Starting from commercially available protected Asp or Glu, the side-chain free carboxylic acid chain is activated by treatment with carbonyldiimidazole. This activated ester is subsequently subjected to condensation with the magnesium salt of monoethyl malonic acid. The magnesium salt of monoethyl malonic acid is preferably prepared from commercial potassium monoethyl manolate, through acidification at low temperature and treatment with magnesium ethoxide. Pure -ketoesters, also named compound (1) throughout the present specification, are obtained with good yields after column chromatography on silica-gel.

Accordingly, examples of the compound (1) include (2S)-2-Benzyloxycarbonylamino-4-oxo-hexanedioic acid 1-benzyl ester 6-ethyl ester, (2R)-2-tert-Butoxycarbonylamino-4-oxo-hexanedioic acid 1-benzyl ester 6-ethyl ester and (2S)-2-Benzyloxycarbonyl-amino-5-oxo-heptanedioic acid 1-benzyl ester 7-ethyl ester.

For preparing fluorescent polypeptides containing, in their amino acid chain, one or more fluorescent coumaryl amino acid derivatives according to the invention, notably when performing solid phase peptide synthesis, it is desirable that said coumaryl amino acid derivatives are protected on their amino- or carboxy- reactive groups before using them in the solid phase synthesis chemical reactions. Consequently, in a preferred embodiment of the method according to the invention, the coumaryl amino acid derivatives of formula (I) are further protected on their amino- or carboxy- reactive groups by carrying out further steps following step (b) or (b2) of the method.

Thus, the present invention also pertains to a method for manufacturing an optically pure coumaryl amino acid that is protected on its amino group, wherein said method comprises the steps of :
(A) obtaining the compound of formula (I) according to the method of any one of claims 1 to 4; and (c) reacting the compound of formula (I) obtained at step (A) with the appropriate protective group, whereby obtaining the protected compound of formula (II) : 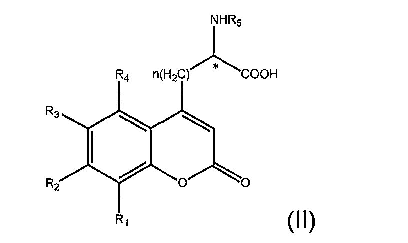
wherein R₅ is a protective group.

Most preferably, the protective group R₅ is selected from the group consisting of Fmoc (9-fluorenylmethylcarbonyl), Cbz (benzyloxycarbonyl), Boc (tert-butyloxycarbonyl), Troc (trichloroethylcarbonyl), Phth (phtalimide), Ac (acetyl), Trifluoroacetyl, Benzoyl, For (formyl), For (formyl), iBut (isobutyryl), Pam (palmytoyl), Trt (trityl), Phenyl, Methyl.

According to the method above, coumaryl amino acids obtained in step (c) are protected on their amino function using classically used protecting groups such as Fmoc, Cbz or Boc. Protected coumarin-bearing amino acids described in the formula (II), also called compound (4), were obtained in good yield after purification by flash chromatography on silica gel or semi-preparative HPLC, for example by using a gradient H₂O + TFA2 (0.1%)/H₂O: CAN (30:70) + TFA (0.09%).

In the above-described compound (4), n and * are as defined in formula (II) ; R₁, R₂, R₃, R₄ and R₅ have the same meaning as defined in formula (II).

Accordingly, examples of the compound (4) include (2S)-2-Fmoc-amino-3-(7-methoxy-2-oxo-2H-chromen-4-yl)propionic acid, (2S)-2-Cbz-amino-3-(7-methoxy-2-oxo-2H-chromen-4-yl)propionic acid, (2S)-2-Boc-amino-3-(7-methoxy-2-oxo-2H-chromen-4-yl)propionic acid and (2R)-2-Fmoc-amino-3-(7-methoxy-2-oxo-2H-chromen-4-yl)propionic acid.

The present invention also relates to a method for manufacturing an optically pure coumaryl amino acid that is protected on its carboxyl group, wherein said method comprises the steps of :
(A) obtaining the compound of formula (I) according to the method of any one of claims 1 to 4; and
(d) reacting the compound of formula (I) obtained at step (A) with the appropriate protector group, whereby obtaining the protected compound of formula (III) : 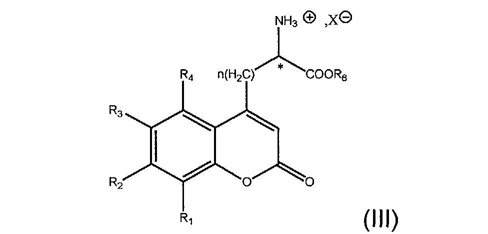 wherein R₆ is a protective group. Most preferably, the protective group R₆ is selected from the group consisting of methyl-, ethyl-, *tert*-butyl-, cyclohexyl-, benzyl- or allyl- ester.

According to the method above, coumaryl amino acids obtained in step (d) are protected on their carboxyl function using classically used protecting groups such as methyl-, ethyl-, *tert*-butyl-, cyclohexyl-, benzyl-or allyl-ester. Protected coumaryl amino acids described in the formula (III), also called compound (5), were obtained in good yields after purification by flash chromatography on silica gel or semi-preparative HPLC, for example by using a gradient H₂O + TFA (0.1%)/H₂O: ACN (30:70) + TFA (0.09%).

In the above-described compound (5), n and * are as defined in formula (II) ; X, R₁, R₂, R₃, R₄ and R₅ have the same meaning as defined in formula (III).

Accordingly, examples of the compound (5) include (1S)-1-benzyloxycarbonyl-3-(7-methoxy-2-oxo-2H-chromen-4-yl)-propyl ammonium trifluoro-acetate.

The protected compounds of formula (II) and (III) above can be readily used in peptide synthesis methods for the purpose of incorporating one or more coumaryl amino acid derivative(s) of the invention within the amino acid chain which is synthesised, at the desired amino acid position(s).

A further object of the invention consists of an optically active coumaryl amino acid salt having the following formula (I) : 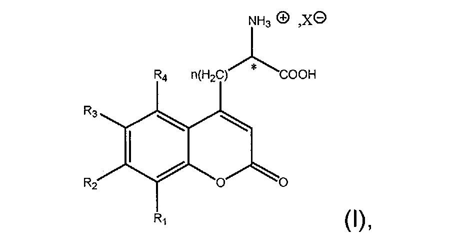 wherein :
(i) n is an integer ranging from 1 to 2 ;
(ii) X⁻ represents Cl⁻, Br⁻, I⁻, CH₃SO₃⁻, CF₃CO₂⁻, CF₃SO₃⁻ ;
(iii) R₁ represents H, halogen, alkyl, acyl, nitrile, sulfonate, aminosulfonyl, carbonyl and carbamoyl, OH, O- or N- substituted by alkyl or acyl group ;
(iv) R₂ represents H, halogen, alkyl, acyl, nitrile, sulfonate, aminosulfonyl, carbonyl and carbamoyl, OH, O- or N- substituted by alkyl or acyl group ;
(v) R₃ represents H, halogen, alkyl, acyl, nitrile, sulfonate, aminosulfonyl, carbonyl and carbamoyl, OH, O- or N- substituted by alkyl or acyl group ;
(vi) R₄ represents H, halogen, alkyl, acyl, nitrile, sulfonate, aminosulfonyl, carbonyl and carbamoyl, OH, O- or N- substituted by alkyl or acyl group ; and
(vii) "*" represents the position of an asymmetric carbon atom;

Preferred optically active coumaryl amino acid salt as defined above are those which are selected from the group consisting of:
- (1 S)-1-carboxy-2-(7-hydroxy-2-oxo-2H-chromen-4-yl)-ethyl ammonium trifluoroacetate,
- (1S)-1-carboxy-2-(7-methoxy-2-oxo-2H-chromen-4-yl)-ethyl ammonium methanesulfonate,
- (1 R)-1-carboxy-2-(7-methoxy-2-oxo-2H-chromen-4-yl)-ethyl ammonium trifluoroacetate,
- (1S)-1-carboxy-2-(6-chloro, 7-hydroxy-2-oxo-2H-chromen-4-yl)-ethyl ammonium trifluoroacetate,
- (1S)-1-carboxy-2-(7-ethoxy-2-oxo-2H-chromen-4-yl)-ethyl ammonium trifluoroacetate,
- (1S)-1-carboxy-2-(5-hydroxy, 7-methoxy-2-oxo-2H-chromen-4-yl)-ethyl ammonium trifluoroacetate,
- (1S)-1-carboxy-2-(7-hydroxy, 5-methoxy-2-oxo-2H-chromen-4-yl)-ethyl ammonium trifluoroacetate,
- (1S)-1-carboxy-2-(5,7-dimethoxy-2-oxo-2H-chromen-4-yl)-ethyl ammonium trifluoroacetate, and
- (1S)-1-carboxy-2-(5,7-dihydroxy-2-oxo-2H-chromen-4-yl)-ethyl ammonium trifluoroacetate.

In a preferred embodiment of a compound of formula (II), groups R1, R2, R3 and R4 are as defined for the compound of formula (I) and group R5 consists of a protective group convention ally used in the art for protecting an amino group. Most preferably, the protective group R5 is selected from the group consisting of :
Fmoc (9-fluorenylmethylcarbonyl), Cbz (benzyloxycarbonyl), Boc (tert-butyloxycarbonyl), Troc (trichloroethylcarbonyl), Phth (phtalimide), Ac (acetyl), Trifluoroacetyl, Benzoyl, For (formyl), iBut (isobutyryl), Pam (palmytoyl), Trt (trityl), Phenyl, Methyl.

The present invention also relates to a compound of formula (II) as defined above, which is selected from the group consisting of :
- (2S)-2-Fmoc-amino-3-(7-methoxy-2-oxo-2H-chromen-4-yl)propionic acid,
- (2S)-2-Cbz-amino-3-(7-methoxy-2-oxo-2H-chromen-4-yl)propionic acid,
- (2S)-2-Boc-amino-3-(7-methoxy-2-oxo-2H-chromen-4-yl)propionic acid; and
- (2R)-2-Fmoc-amino-3-(7-methoxy-2-oxo-2H-chromen-4-yl)propionic acid.

In a preferred embodiment of a compound of formula (III), "n", "*", X, R1, R2, R3 and R4 have the same meaning as for the compound of formula (I) and R5 is selected from the group consisting of methyl, ethyl, *tert*-butyl, cyclohexyl, benzyl or allyl.

The present invention also pertains to a compound of formula (III) as defined above, which consists of (1S)-1-benzyloxycarbonyl-3-(7-methoxy-2-oxo-2H-chromen-4-yl)-propyl ammonium trifluoro-acetate.

A further object of the invention consists of an intermediate compound of formula (1) as defined above, which is useful for the synthesis of a compound of formula (I), wherein said compound of formula (1) is selected from the group consisting of:
- (2S)-2-Benzyloxycarbonylamino-4-oxo-hexanedioic acid 1-benzyl ester 6-ethyl ester,
- (2R)-2-Butoxycarboxylamino-4-oxo-hexanedioic acid 1-benzyl ester 6-ethyl ester; and
- (2S)-2-Benzyloxycarbonyl-amino-5-oxo-heptanedioic acid 1-benzyl ester 7-ethyl ester.

The invention also pertains to an intermediate compound of formula (2) as defined above, useful for the synthesis of a compound of formula (I), wherein said compound of formula (2) is selected from the group consisting of:
- (1S)-1-carboxy-4-ethoxycarbonyl-3-oxo-butyl ammonium chloride; and
- (1R)-1-carboxy-4-ethoxycarbonyl-3-oxo-butyl ammonium chloride.

In example 22 herein, the synthesis of optically active polypeptides is carried out, using a coumaryl amino acid derivative according to the invention.

The present invention also deals with a kit for manufacturing a fluorescent polypeptide, wherein said kit comprises one coumaryl amino acid derivative selected from the compounds of formula I, II and III as defined in the present specification.

Preferably, such a kit comprises a glass vial containing a coumaryl amino acid derivative of the invention in the form of a powder or alternatively under the form of a liquid solution, for example a liquid conventional buffer solution, wherein said amino acid derivative is dissolved or suspended.

Another object of the invention consists of a method for the synthesis of an optically active polypeptide wherein said method comprises at least one step of incorporating an optically active coumaryl amino acid of formula (I) within the amino acid chain.

A further object of the invention consists of an optically active polypeptide which contains in its amino acid chain at least one optically active coumaryl amino acid of formula (I). Such optically active polypeptide may contain more than one coumaryl amino acid derivative in its amino acid chain for example from 2 to 10 of such derivatives, depending on the amino acid length of said optically active polypeptide.

Still further, this invention also relates to an *in vitro* assay kit comprising an optically active polypeptide which contains in its amino acid chain an optically active coumaryl amino acid of formula (I).

The present invention will be further illustrated by, without in any way being limited to, the examples hereunder.

### EXAMPLES

Examples 1 to 5 are intermediate compounds useful for the synthesis of the novel fluorescent amino acids of the invention.

### Intermediate compounds.

### Example 1

### Preparation of (2S)-2-Benzyloxycarbonylamino-4-oxo-hexanedioic acid 1-benzyl ester 6-ethyl ester.

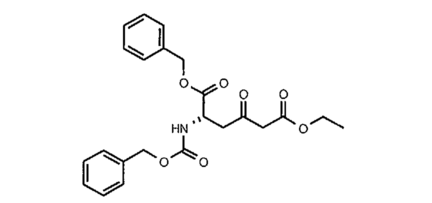

According to the method described by Brooks, to a solution of the Cbz-(L)-Asp-OBn (7.41 g, 20.74 mmol) in THF (4 mL/mmol) carbonyldiimidazole (3.7 g, 22.82 mmol, 1.1 equiv) was added at room temperature. The resulting reaction mixture was stirred two hours at room temperature, then cooled at 0°C before addition of magnesium bis(monoethylmanolate) (3.21 g, 11.20 mmol, 0.54 equiv). The reaction mixture was stirred overnight at room temperature as slow CO₂ release was observed. It was taken up in ether (15-20 mL/mmol of amino acid) and acidified with concentrated HCl (1 N or 6N) at 0°C ; the ethereal extract was washed with 10% NaHCO₃, 4:1 H₂O/KHSO₄(1M), H₂O and brine, dried (Na₂SO₄) and concentrated *in vacuo*. The residue was purified by flash chromatography on silica gel (1: 1 EtOAc/cyclohexan), leading to example (1) (6.83 g, 16.0 mmol, 77%) as a white solid.

¹H-NMR (400 MHz, CDCl₃) : 1.26 (t, 3H, J = 7.2), 3.15 (dd, 1 H, J = 18.6, 4.2), 3.32 (dd, 1 H, J = 18.6, 4.2), 3.42 (s, 2H), 4.17 (q, 2H, J = 7.2), 4.52-4.71 (m, 1 H), 5.12 (s, 2H), 5.17 (s, 2H), 5.78 (d, 1 H, J = 8.4), 7.33 (m, 10H).

¹³C-NMR (CDCl₃) : 13.7, 44.2 , 48.7 , 49.5 , 61.2 , 66.7 and 67.2, 127.7, 127.8, 127.8, 128.0, 128.1 and 128.2 , 134.8 and 135.7, 155.6, 166.1, 170.2 and 200.4.

### Example 2

### Preparation of (2R)-2-tert. Butoxycarbonylamino 4-oxo-hexanedioic acid 1-benzyl ester 6-ethyl ester

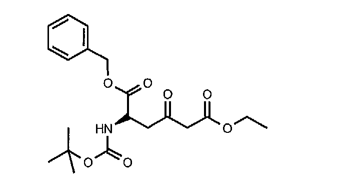

Following the same procedure as for example (1) from Boc-(D)-Asp-OBn (2 g, 6.2 mmol), example (2) (1.609 g, 4.1 mmol, 66%) was obtained as a pure white solid.

¹H-NMR (400 MHz, CDCl₃): 1.27 (t, 3H, J = 7.2), 1.45 (s, 9H), 3.12 (dd, 1 H, J = 18.4, 4.4), 3.30 (dd, 1 H, J = 18.4, 4.4), 3.43 (s, 2H), 4.14-4.22 (q, 2H, J = 7.2 ), 4.58 (m, 1 H), 5.16 (s, 2H), 5.48 (d, 1 H, J = 8.4 ), 7.3-.74 (m, 5H).

### Example 3

### Preparation of (2S)-2-Benzyloxycarbonylamino-5-oxo-heptanedioic acid 1-benzyl ester 7-ethyl ester

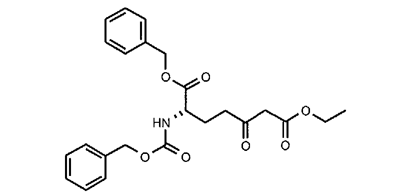

Following the same procedure as for example (1) from Cbz-(L)-Glu-OBn (10 g, 26.93 mmol), example (3) (6.187 g, 14 mmol, 52%) was obtained as a pure white solid.

¹H-NMR (400 MHz, CDCl₃) : 1.26 (t, 3H, J = 7.2), 1.9-2.01 (m, 1 H), 2.1-2.28 (m, 1 H), 2.5-2.7 (m, 2H), 3.37 (s, 2H), 4.18 (q, 2H, J = 7.2), 4.41 (m, 1H), 5.11 (s, 2H), 5.18 (s, 2H), 5.44 (d, 1 H, J = 8), 7.25-7.41 (m, 10H).

### Example 4

### Preparation of (1S)-1-carboxy-4-ethoxycarbonyl-3-oxo-butylammonium chloride

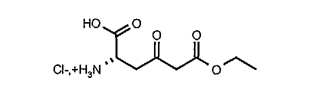

The -ketoester example (1) (6.78 g, 15.8 mmol) was dissolved in 1:1 AcOEt/ 95% EtOH and HCl 1 N (1 equiv) and subjected to hydrogenolysis under atmospheric pressure over 10% Pd on charcoal (0.05 equiv). The resulting reaction mixture was filtrated through celite, washed with 95% EtOH and concentrated *in vacuo*. The residue was taken up in water and lyophilised leading to example (4) (3.72 g, 15.5 mmol, 97%) as a pure white solid.

¹H-NMR (400 MHz, DMSO) : 1.14 (t, 3H, J = 7.2), 3.13 (dd, 2H, J = 18.8, 5.2), 3.21 (dd, 2H, J = 18.8, 5.2), 3.62 (d, 1 H, J = 16.4), 3.67 (d, 1 H, J = 16.4), 4.05 (q, 2H, J = 7.2), 4.09-4.15 (m, 1 H), 8.40 (s, 3H).

¹³C-NMR (DMSO) : 14.0 (CH₃), 42.0 (CH₂), 47.3 and 48.7 (CH and CH₂ -ketoester), 60.7 (CH₂), 166.7 (C=O ester), 170.0 (C=O acid), 200.1 (C=O ketone).

### Example 5

### Preparation of (1R)-1-carboxy-4-ethoxycarbonyl-3-oxo-butylammonium chloride

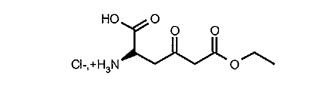

Following the same procedure as for example (4) from example (2) (1.43 g, 3.6 mmol), the benzyl group was removed and the resulting acid was treated for removal of the Boc group in a mixture of 1:1 CHCl₃/TFA (24mL) added at 0°C. After one hour at room temperature, the mixture was concentrated *in vacuo*, the residue taken up in water and lyophilised giving example (5) as a pure solid (1g, 3.3 mmol, 91 %).

¹H-NMR (400 MHz, DMSO) : 1.15 (t, 3H, J = 7.2 ), 3.09 (dd, 1H, J = 19, 6), 3.16 (dd, 1 H, J = 19, 4.4), 3.65 (s, 2H), 4.05 (q, 2H, J = 7.2), 4.11-4.21 (m, 1 H), 8.16 (s, 3H).

### Fluorescent amino acid compounds of the invention

### Example 6 (Compound (C1)

### Preparation of (1S)-1-carboxy-2-(7-hydroxy-2-oxo-2H-chromen-4-yl)-ethvl ammonium trifluoroacetate

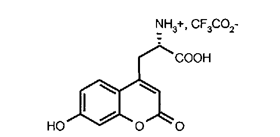

Amino acid derived -ketoester example (4) (197 mg, 0.82 mmol) and resorcinol (136 mg, 1.24 mmol) were mixed before addition, at 0°C, of 99% methanesulfonic acid (25 equiv) and stirred at room temperature for two hours. The deep-red, homogenous reaction mixture was taken up in cold ether (-30°C) and centrifuged 20 minutes at 3600 g. After ether discarding, the residue was washed once more with cooled ether, centrifuged 20 minutes at 3200 rpm, taken up in water and lyophilised to give example 6 (97 mg, 0.39 mmol, 47%) as a pale yellow solid.

¹H-NMR (400 MHz, DMSO) : 3.08 (dd, 1H, J = 15.2, 6.8), 3.32 (dd, 1 H, J = 15.2, 5.2), 4.12-4.22 (m, 1 H), 6.16 (s, 1 H), 6.71 (d, 1 H, J = 2.4), 6.79 (dd, 1 H, J = 8.8, 2.4), 7.56 (d, 1 H, J = 8.8), 8.28 (s, 3H), 10.63 (s, 1 H).

¹³C-NMR (400 MHz, DMSO) : 32.0 (I), 51.0 (k), 102.6, 110.7, 112.8, 113.1, 126.0, 149.6, 155.3, 160.0, 161.4, 170.0.

Optical rotation, determined with a Jasco model P-1030 polarimeter : [ ]_{D} = 8.4 (c = 0.485, 1 N HCl, 20°C).

### Example 7- Compound (C2).

### Preparation of (1 S)-1-carboxy-2-(7-methoxy-2-oxo-2H-chromen-4-yl)-ethyl ammonium methanesulfonate

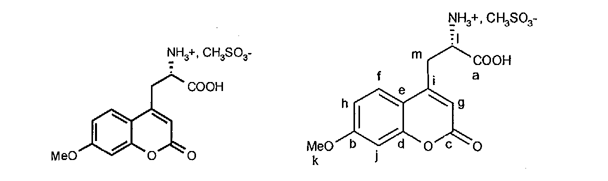

Following the same procedure as for example (6) from example (4) (1.786 g, 7.45 mmol) and 3-methoxyphenol (1.387 g, 11.18 mmol), example (7) (1.9 g, 5.3 mmol, 71%) was obtained as a pure white powder.

¹H-NMR (400 MHz, DMSO) : 2.31 (s, 3H), 3.12 (dd, 1 H, J = 14.4, 8.8), 3.35 (dd, 1 H, J = 14.4, 5.8), 3.82 (s, 3H), 4.11-4.23 (m, 1 H), 6.25 (s, 1 H), 6.96 (dd, 1 H, J = 8.8, 2.4), 6.70 (d, 1 H, J = 2.4), 7.66 (d, 1 H, J = 8.8), 8.31 (s, 3H).

¹³C-NMR (DMSO) 31.9 (m), 51.0 (l), 56.1 (k), 101.2 (j), 111.9 (i), 112.4 (h), 113.8 (g), 126.0 (f), 149.7 (e), 155.3(d), 159.9 (c ), 162.6 (b) and 167.0 (a).

Optical rotation, determined with a Jasco model P-1030 polarimeter : [ ]_{D} = 12.9 (c = 0.7, 1 N HCl, 20°C).

### Example 8 ― Compound (C3).

### Preparation of (1 R)-1-carboxy-2-(7-methoxy-2-oxo-2H-chromen-4-yl)-ethyl ammonium trifluoroacetate

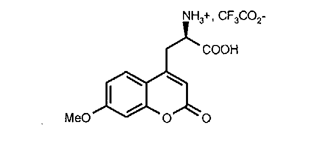

Following the same procedure as for example (6) from example (5) (694 mg, 2.9 mmol) and 3-methoxyphenol (540 mg, 4.34 mmol), example (8) (728 mg, 2.03 mmol, 70%) was obtained as a pale yellow solid after purification by semi-preparative HPLC.

¹H-NMR (400 MHz, DMSO) : 2.99 (dd, 1 H, J = 14.6, 11.2), 3.39 (dd, 1 H, J = 14.6, 4.6), 3.81 (s, 3H), 3.85-3.98 (m, 1 H), 6.22 (s, 1H), 6.96 (dd, 1 H, J = 8.8, 2.4), 7.00 (d, 1 H, J = 2.4), 7.66 (d, 1 H, J = 8.8), 8.12 (s, 3H).

Optical rotation, determined with a Jasco model P-1030 polarimeter : [ ]_{D} = -12.07° (c = 0.468, 1 N HCl, 20°C).

### Example 9 ― Compound (C9).

### Preparation of (1S)-1-ethoxycarbonyl-2-(6,7-dimethoxy-2-oxo-2H-chromen-4-yl)-ethyl ammonium trifluoroacetate

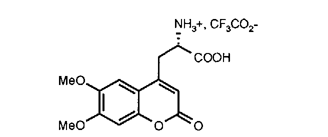

Following the same procedure as for example (6) from example (4) (556 mg, 2.32 mmol) and 3,4-dimethoxyphenol (537 mg, 3.48 mmol), example (9) (426 mg, 1.04 mmol, 45 %) was obtained as a pure pale yellow solid.

¹H-NMR (400 MHz, DMSO): 3.15 (dd, 1H, J = 14.4, 8.8), 3.35 (dd, 1 H, J = 14.4, 5.6), 3.80 (s, 3H), 3.81 (s, 3H), 4.22-4.35 (m, 1 H), 6.24 (s, 1 H), 7.01-7.75 (m, 1 H), 7.10 (s, 1 H), 8.31 (s, 3H).

¹³C-NMR (400 MHz, DMSO + TFA) : 31.9, 50.9, 56.0, 56.3, 100.5, 105.7, 113.8, 115.1 (q, J = 1148), 145.8, 149.3, 149.6, 152.7, 158.4 (q, J = 152), 160.1, 170.2.

### Example 10- Compound (C4).

### Preparation of (1S)-1-carboxy-2-(6-chloro, 7-hydroxy-2-oxo-2H-chromen-4-yl)-ethyl ammonium trifluoroacetate

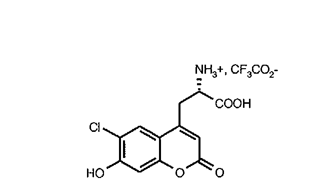

Following the same procedure as for example (6) from example (4) (516 mg, 3.57 mmol) and 4-chloro-resorcinol ( 515.7 g, 3.57 mmol), example (10) (50 mg, 0.13 mmol, 16%) was obtained as a pure white solid.

¹H-NMR (400 MHz, DMSO) : 3.07 (dd, 1H, J = 14.4, 8.8), 3.3 (dd, 1 H, J = 14.4, 5.6), 4.16-4.28 (m, 1 H), 6.23 (s, 1 H), 6.89 (s, 1 H), 7.73 (s, 1 H), 8.3 (s, 3H).

¹³C-NMR (400 MHz, DMSO) : 32.4, 51.9, 103.7, 111.6, 113.5, 117.1, 125.6, 150.1, 153.5, 156.7, 159.7, 169.7.

Optical rotation, determined with a Jasco model P-1030 polarimeter : [ ]_{D} = 3.23° (c = 0.384, EtOH 95%, 20°C).

### Example 11 Compound (C5).

### Preparation of (1 S)-1-carboxy-2-(7-ethoxy-2-oxo-2H-chromen-4-yl)-ethyl ammonium trifluoroacetate (6).

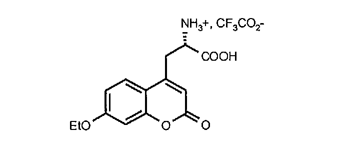

Following the same procedure as for example (6) from example (4) (547 mg, 2.3 mmol) and 3-ethoxyphenol (477 mg, 3.45 mmol), example (11) (341 mg, 0.9 mmol, 39%) was obtained as a pure white solid.

¹H-NMR (400 MHz, DMSO) : 1.31 (t, 3H, J = 6.8), 3.07 (dd, 1H, J = 14.4, 9.6), 3.35 (dd, 1 H, J = 14.4, 5.2), 4.09 (q, 2H, J = 6.8), 4.12-4.24 (m, 1H), 6.20 (s, 1 H), 6.9-6.99 (m, 2H), 7.65 (d, 1 H, J = 8.8), 8.28 (s, 3H).

¹³C-NMR (400 MHz, DMSO + TFA) : 14.3, 31.9, 50.9, 64.1, 101.5, 111.7, 112.6, 115.1 (q, J = 1147), 125.8, 149.5, 155.3, 158.5 (q, J = 152), 159.9, 161.8, 170.0.

Optical rotation, determined with a Jasco model P-1030 polarimeter : [ ]_{D} = 15.7° (c = 0.476, 1 N HCl, 20°C).

### Examples 12 and 13 ― Compounds (C10) and (C6).

### Preparation of (1S)-1-carboxy-2-(5-hydroxy, 7-methoxy-2-oxo-2H-chromen-4-yl)-ethyl ammonium trifluoroacetate (10) and (1S)-1-carboxy-2-(7-hydroxy, 5-methoxy-2-oxo-2H-chromen-4-yl)-ethyl ammonium trifluoroacetate

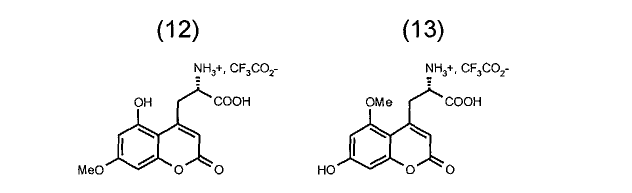

Following the same procedure as for example (6) from example (4) (536 mg, 2.24 mmol) and 5-methoxyresorcinol (471 mg, 3.36 mmol), example (12) [compound (10)] (300 mg, 0.8 mmol, 36%) and example 13 [compound (6)] (120 mg, 0.4 mmol, 18%) were obtained in a 2 : 1 proportion as pure pale yellow solids after separation by semi-preparative HPLC.

Example 12 : ¹H-NMR (400 MHz, DMSO) : 2.58-2.71 (m, 1H), 3.55-3.68 (m, 1 H), 3.72-3.8 (m, 1 H), 3.81 (s, 3H), 5.90 (s, 1 H), 6.30 (d, 1H, J = 2), 6.34 (d, 1H, J = 2), 7.65 (broad single, 3H), 10.64 (s, 1H).

¹³C-NMR (400 MHz, DMSO + TFA) : 36.9, 51.6, 56.0, 95.8, 96.0, 101.6,113.1,115.1 (q, J = 1147), 150.0, 156.8, 158.5 (q, J = 152), 159.6, 161.8, 170.5.

Example 13 : ¹H-NMR (400 MHz, DMSO) : 2.80(dd, 1 H, J = 12.8, 10.8), 3.73 (s, 3H), 3.81 (dd, 1 H, J = 12.8, 4.0), 3.94-4.09 (m, 1H), 3.81 (s, 3H), 5.96 (s, 1 H), 6.30 (d, 1 H, J = 2.4), 6.44 (d, 1 H, J = 2.4), 7.9 (broad single, 3H).

Optical rotation, determined with a Jasco model P-1030 polarimeter : [ ]_{D} = 10.1 ° (c = 0.323, EtOH 95%, 20°C).

### Example 14 -Compound (C7)

### Preparation of (1S)-1-carboxy-2-(5,7-dimethoxy-2-oxo-2H-chromen-4-yl)-ethyl ammonium trifluoroacetate

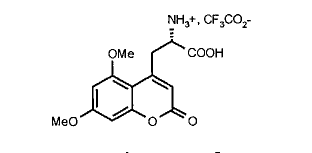

Following the same procedure as for example (6) from example (4) (547 mg, 2.28 mmol) and 3,5-dimethoxyphenol (528 mg, 3.42 mmol), example (14) (316 mg, 0.78 mmol, 34 %) was obtained as a pure a pure white solid.

¹H-NMR (400 MHz, DMSO) : 2.84 (dd, 1 H, J = 12.8, 10.8), 3.73 (dd, 1 H, J = 12.8, 3.8), 3.81 (s, 3H), 3.85 (s, 3H), 3.99-4.11 (m, 1 H), 6.01 (s, 1 H), 6.51 (d, 1 H, J = 2.0), 6.61 (d, 1 H, J = 2.0), 8.17 (s, 3H).

¹³C-NMR (400 MHz, DMSO) : 36.9, 51.6, 56.0, 56.3, 94.0, 95.6, 102.8, 114.1, 149.7, 156.8, 159.5, 162.9, 170.4.

Optical rotation, determined with a Jasco model P-1030 polarimeter : [ ]_{D} = 12.0° (c = 0.472, EtOH 95%, 20°C).

### Example 15 ― Compound (C8)

### Preparation of (1 S)-1-carboxy-2-(5,7-dihydroxy-2-oxo-2H-chromen-4-yl)-ethyl ammonium trifluoroacetate

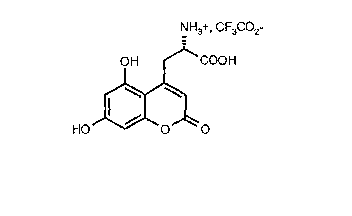

Following the same procedure as for example 6 from example 4 (553 mg, 2.31 mmol) and 3,5-dihydroxyphenol (561 mg, 3.46 mmol), example (15) (420.2 mg, 1.1 mmol, 48%) was obtained as a pure pale yellow powder.

¹H-NMR (DMSO) : 2.80 (dd, 1 H, J = 13, 11.6), 3.78 (dd, 1 H, J = 13, 4), 4.05-4.2 (m, 1 H), 5.88 (s, 1 H), 6.17 (d, 1 H, J = 2.2), 6.25 (d, 1 H, J = 2.2), 8.16 (s, 3H), 10.40 (s, 1 H), 10.98 (s, 1 H).

Optical rotation, determined with a Jasco model P-1030 polarimeter : [ ]_{D} = 13.2° (c = 0.448, 1 N HCl, 20°C).

### Examples 16 and 17

### Preparation of (1 S)-1-carboxy-3-(7-methoxy-2-oxo-2H-chromen-4-yl)-propyl ammonium trifluoroacetate and (1S)-1-benzyloxycarbonyl-3-(7-methoxy-2-oxo-2H-chromen-4-yl)-propyl ammonium trifluoroacetate

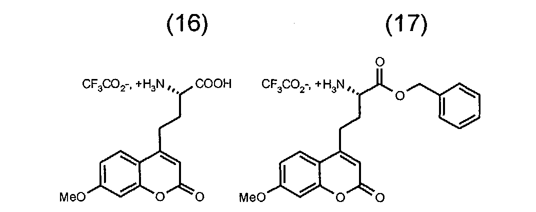

Following the same procedure as for example (6) from example (3) (300 mg, 0.7 mmol) and 3-methoxyphenol (869 mg, 7 mmol), example (16) (98 mg, 0.26 mmol, 37%) and example (17) (76 mg, 0.16 mmol, 23%) were obtained in 62 : 38 proportion as a pure orange solid and a pure white solid respectively after separation by semi-preparative HPLC.

Example 16 : ¹H-NMR (400 MHz, DMSO) : 1.92-2.16 (m, 2H), 2.72-2.97 (m, 2H), 3.82 (s, 3H), 3.82-4.0 (m, 1H), 6.16 (s, 1 H), 6.95 (dd, 1 H, J = 8.8, 2.4), 6.98 (d, 1 H, J = 2.4), 7.68 (d, 1 H, J = 8.8), 8.17 (s, 3H).

¹³C-NMR (400 MHz, D₂O) : 29.6, 31.2, 55.5, 58.7, 103.8, 112.5, 115.1, 115.8, 128.5, 157.4, 159.7, 165.4, 167.4, 174.8.

Optical rotation, determined with a Jasco model P-1030 polarimeter : [ ]_{D} = 19.4° (c = 0.504, 1 N HCl, 20°C).

Example 17 : ¹H-NMR (DMSO) : 1.88-2.11 (m, 2H), 2.68-2.89 (m, 2H), 3.81 (s, 3H), 3.73-4.02 (m, 1H), 5.17 (s, 2H), 6.10 (s, 1H), 6.88 (dd, 1 H, J = 8.8, 2.4), 6.96 (d, 1 H, J = 2.4), 7.23-7.42 (m, 5H), 7.57 (d, 1H, J = 8.8).

¹³C-NMR (400 MHz, DMSO) : 26.5, 28.7, 51.5, 56.0, 67.4, 101.1, 110.6, 111.9, 112.3, 125.9, 128.5, 128.6, 135.1, 154.7, 155.1, 160.1, 162.5, 169.2.

### Example 18

### Preparation of (2S)-2-(Fmoc-amino)-3-(7-methoxy-2-oxo-2H-chromen-4-yl)propionic acid

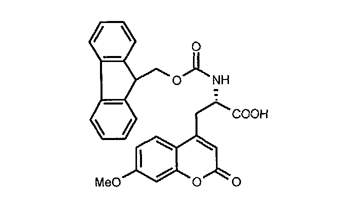

The example (7) coumaryl amino acid (510 mg, 1.42 mmol) dissolved in 1: 1 dioxane/H₂O (7 mL/mmol) was treated at 0°C with 10% NaHCO₃. One minute later, FmocCl (404 mg, 1.57 mmol, 1.1 equiv) was added at 0°C ; the reaction mixture was stirred on hour at 0°C then two hours at room temperature. The reaction mixture was taken up in AcOEt, the organic extract was washed with H₂O, 1 N HCl and brine, dried (Na₂SO₄) and concentrated *in vacuo*. The residue was purified by flash chromatography on silica gel (4: 1 EtOAc/cyclohexane ― pure AcOEt) to give a pure white powder.(513 mg, 1.14 mmol, 80%).

¹H-NMR (DMSO) : 3.00 (dd, 1 H, J = 14.4, 11.2), 3.28 (dd, 1 H, J = 14.4, 3.4), 3.80 (s, 3H), 4.13 (t, 1 H, J = 6.4), 4.17 (d, 2H, J = 6.4), 4.2-4.31 (m, 1 H), 6.20 (s, 1 H), 6.95 (dd, 1 H, J = 8.8, 2.4), 6.98 (d, 1 H, J = 2.4), 7.26 (dd, 2H, J = 14.4, 7.2), 7.35 (t, 2H, J = 7.6), 7.57 (dd, 2H, J = 11.8, 7.6), 7.7 (d, 1 H, J = 8.8), 7.83 (dd, 2H, J = 8.0, 3.6).

¹³C-NMR (400 MHz, DMSO): 21.1, 32.7, 46.5, 52.8, 56.0, 66.8, 66.4, 101.1, 112.1, 112.4, 120.2, 125.15, 125.23, 125.9, 127.1, 127.7, 140.7, 143.67, 143.74, 152.7, 155.1, 156.0, 160.0, 162.4, 172.7.

### Example 19

### Preparation of (2S)-2-(Cbz-amino)-3-(7-methoxy-2-oxo-2H-chromen-4-yl)propionic acid

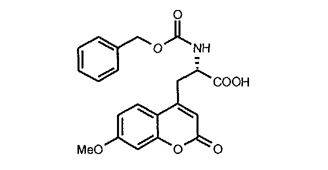

To a cold solution of NaOH (66 mg, 1.66 mmol, 2 equiv) in 1: 1 dioxane/H₂O (2.7 mL) was added the example (7) coumaryl amino acid (300 mg, 0.83 mmol) at 0°C. A solution of benzyle chloroformiate (157 mg, 0.92 mmol, 1.1 equiv) in dioxane (0.4 mL) was carefully added at 0°C (pH 9-11). After one hour at room temperature, the reaction mixture was acidified with 1 N HCl and extracted with AcOEt (40mL). The organic extract was washed with brine, dried (Na₂SO₄) and concentrated *in vacuo* giving the example (19) (300 mg, 0.75 mmol, 91%) as a pure pale brown powder.

¹H-NMR (DMSO) : 2.97(dd, 1 H, J = 14.4, 10.8), 3.26(dd, 1 H, J = 14.4, 3.6), 3.77 (s,3H), 4.22-4.32 (m,1H), 4.91 (s, 2H), 6.15 (d, 1 H), 6.95 (dd, 1 H, J = 8.8, 2.8), 6.97 (d, 1 H, J = 2.8), 7.09-7.4 (m, 5H), 7.69 (d, 1 H, J = 8.8), 7.75 (d, 1 H, J = 8.0).

¹³C-NMR (400 MHz, DMSO + TFA) : 32.8, 52.8, 56.0, 65.4, 101.1, 112.1, 112.4, 125.9, 127.4, 127.8, 128.0, 128.4, 136.9, 152.6, 155.1, 156.0, 159.9, 162.4, 172.5.

### Example 20

### Preparation of (2S)-2-(Boc-amino)-3-(7-methoxy-2-oxo-2H-chromen-4-yl)propionic acid

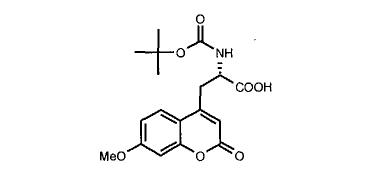

The example (7) coumaryl amino acid (300 mg, 0.83 mmol) in dioxane (3.5 mL) is treated by 5% NaHCO₃ (3.5 mL, 175 mg, 2.08 mmol). At 0°C was added Boc₂O (218 mg, 1 mmol). The resulting reaction mixture was stirred one hour at 0°C and three hours at room temperature, taken up in AcOEt (180 mL) and acidified by 10% citric acid (30 mL). The organic extract was washed with H₂O (3 20 mL) and brine (3 50 mL), dried (Na₂SO₄) and concentrated *in vacuo* giving example (20) ( 254 mg, 0.70 mmol, 84 %) as a pure pale brown oil.

¹H-NMR (DMSO) : 1.25 (s, 9H), 2.82-2.96 (m, 1H), 3.13-3.27 (m, 1 H), 3.81 (s, 3H), 4.1-4.2 (m, 1 H), 6.13 (s, 1 H), 6.9-7.0 (m, 2H), 7.16 (d, 1 H, J = 8.0), 7.68 (d, 1 H, J = 8.4).

¹³C-NMR (400 MHz, DMSO) : 26.9, 27.6, 28.1, 31.3, 33.1, 52.6, 56.0, 78.3, 85.7, 101.1, 112.1, 112.2, 112.4, 125.9, 146.3, 152.9, 155.1, 155.4, 160.0, 162.4, 172.8.

### Example 21

### Preparation of (2R)-2-(Fmoc-amino)-3-(7-methoxy-2-oxo-2H-chromen-4-yl)propionic acid

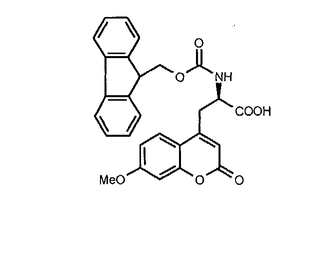

Following the same procedure as for example (18) from example (9) coumaryl amino acid (631 mg, 1.76 mmol), example (21) was obtained, after purification by semi-preparative HPLC and lyophilization, as a pure white powder (556 mg, 1.23 mmol, 70%).

¹H-NMR (DMSO): 3.00 (dd, 1 H, J = 14.4, 11.2), 3.28 (dd, 1 H, J = 14.4, 3.4), 3.80 (s, 3H), 4.13 (t, 1 H, J = 6.4), 4.17 (d, 2H, J = 6.4), 4.2-4.31 (m, 1 H), 6.20 (s, 1 H), 6.95 (dd, 1 H, J = 8.8, 2.4), 6.98 (d, 1 H, J = 2.4), 7.26 (dd, 2H, J = 14.4, 7.2), 7.35 (t, 2H, J = 7.6), 7.57 (dd, 2H, J = 11.8, 7.6), 7.7 (d, 1 H, J = 8.8), 7.83 (dd, 2H, J = 8.0, 3.6).

¹³C-NMR (400 MHz, DMSO): 21.1, 32.7, 46.5, 52.8, 56.0, 66.8, 66.4, 101.1, 112.1, 112.4, 120.2, 125.15, 125.23, 125.9, 127.1, 127.7, 140.7, 143.67, 143.74, 152.7, 155.1, 156.0, 160.0, 162.4, 172.7.
The Physicochemical properties, and more specifically the fluorescence properties of the novel compounds according to the invention are disclosed in Table 1 below.

**Table 1 :**

| **Physicochemical properties** | | | | | |
|---|---|---|---|---|---|
| Compound | _{D}^{a} | Abs. (nm)^{b} | Em. (nm)^{b} | (cm⁻¹ M⁻¹) ^{c} | QY ^{d} |
| W | - | 278 | 352 | 5300 | 0.12 |
| Y | - | 274 | 303 | 1400 | 0.13 |
| 6 | 8.4° | 329 | 464 | 9000 | 0.49 |
| 7 | 12.9° | 324 | 383 | 12000 | 0.18 |
| 8 | -12.1° | 323 | 386 | 14000 | 0.15 |
| 9 | n.d.^{e} | 345 | 445 | 12000 | 0.60 |
| 10 | 3.2° | 331 | 465 | 10300 | 0.54 |
| 11 | 15.7° | 325 | 387 | 13300 | 0.17 |
| 12 | n.d.^{e} | 329 | 421 | 6800 | 0.50 |
| 13 | 10.1° | 323 | 421 | 12700 | 0.18 |
| 14 | 12.0° | 324 | 419 | 11500 | 0.39 |
| 15 | 13.2° | 327 | 463 | 11800 | n.d. ^{e} |
| 16 | 19.4° | 321 | 380 | 12500 | 0.21 |
| 17 | n.d.^{e} | 322 | 381 | 14600 | 0.17 |

| | | | | | |
|---|---|---|---|---|---|
| a: determined in 1 N HCl | | | | | |
| b : determined in 95% ethanol | | | | | |
| c : extinction coefficient | | | | | |
| d : quantum yield | | | | | |
| e : not determined | | | | | |

Synthesis of two fluorescent peptides using the novel fluorescent amino acids of the invention.

### EXAMPLE 22- Synthesis of two fluorescent peptides.

We validate the use of these fluorescent amino acids in SPPS by incorporating compound described as example 18 (Mca) at the C-terminal end of two 16-amino acid long polypeptides derived from the third helix of the Antennapedia homeodomain.

### Peptide 1

H2N-Arg-Gln-Ile-Lys-Ile-Trp-Phe-Gln-Asn-Arg-Arg-Met-Lys-Trp-Lys-Lys-
Mca-COOH (SEQ ID N°1).

### Peptide 2

H₂N-Arg-Gln-Ile-Lys-Ile-Trp-Phe-Pro-Asn-Arg-Arg-Met-Lys-Trp-Lys-Lys-
Mca-COOH (SEQ ID N°2).

### Experimental protocol.

Assembly of the protected peptide chains was carried out using the stepwise solid-phase method of Merrifield on an Applied Blosystems (ABI) 431A peptide synthetizer using ABI small-Fmoc chemistry on an HMP resin and DCC/HOBt as coupling method. Fmoc groups were removed by piperidine (20% in dichloromethane). Both peptides were synthesized by first as coupling the compound of example 18 to the resin.

After total amino acid deprotection, the final peptidyl resin was dried and cleaved with a mixture of TFA/TIPS/H₂O (9.5/0.25/0.25 in volume) for 4 h at room temperature. After partial evaporation, the filtrate from the cleavage reaction was precipitated with cold ether, and the precipitate was collected by centrifugation. The crude peptide was purified by semipreparative HPLC on a Nucleosil C₁₈ column (Vydac, 5 µm, 10 x 250 mm), and the fractions were analysed by analytical HPLC on a nucleosil C₁₈ column (Vydac, 5 µm, 4,6 x 250 mm). The pure fractions were collected and lyophilised. The structure of the peptides was confirmed by electrospray mass (peptide 1: ₁ = 17.1 min, MS (ESI) = 2490,8 ; peptide 2 : ₂ = 17.4 min, MS (ESI) = 2460.0).

### REFERENCES

Von Pechmann H. et al., *Chem. Ber.,* **1884**, 17, 929

Oppolzer W. et al., *Tet. Let.,* **1989**, *30*, 6009.

Perkin : J.R. Johnson, Org. React, 1942, 1, 210.

Knoevenagel : G. Brufola and al., Heterocycles, 1996, 43, 127.

Reformatsky : R.L. Shriner, Org. React. 1942, 1,1

Wittig : I. YAVARI. R. Hekmat-Shoar and A. Zanouzi, Terahedron Lett., 1998, 39, 16, 2391.

William: W. Zhai, Tetrahedron, 1988, 44, 17, 5425-5430.

Bennett F.A. D.J. Barlow, A.N. O. Dodoo, R.C. Hider, A.B. Lansley, M.J. Lawrence, C. Marriott and S.S. Bansal, Tetrahedron Lett., 1997, 38, 42, 7449-7452.

Kele P. , G. Sui, Q. Huo and R.M. Leblanc, Tetrahedron: Asymmetry, 2000,11,4959-4963.

Brooks: D.W. Books, L.D. L. Lu and S. Masamune, Angew. Chem. Int. Ed. Engl., 1979, 18; 72-74.

## Claims

1. A method for manufacturing an optically active coumaryl amino acid salt having the following formula (I) : 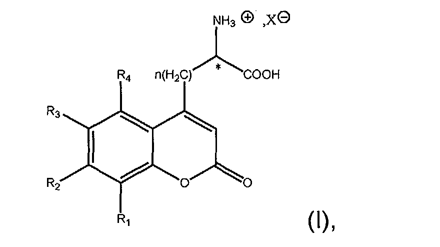 wherein :
(i) n is an integer ranging from 1 to 2 ;
(ii) X⁻ represents Cl⁻, Br⁻, I⁻, CH₃SO₃⁻, CF₃CO₂⁻, CF₃SO₃⁻ ;
(iii) R₁ represents H, halogen, alkyl, acyl, nitrile, sulfonate, aminosulfonyl, carbonyl and carbamoyl, OH, O- or N- substituted by alkyl or acyl group ;
(iv) R₂ represents H, halogen, alkyl, acyl, nitrile, sulfonate, aminosulfonyl, carbonyl and carbamoyl, OH, O- or N- substituted by alkyl or acyl group ;
(v) R₃ represents H, halogen, alkyl, acyl, nitrile, sulfonate, aminosulfonyl, carbonyl and carbamoyl, OH, O- or N- substituted by alkyl or acyl group ;
(vi) R₄ represents H, halogen, alkyl, acyl, nitrile, sulfonate, aminosulfonyl, carbonyl and carbamoyl, OH, O- or N- substituted by alkyl or acyl group ; and
(vii) "*" represents the position of an asymmetric carbon atom;
wherein said method comprises the step of :
(b) reacting the amino acid -ketoester of the following formula (1) : 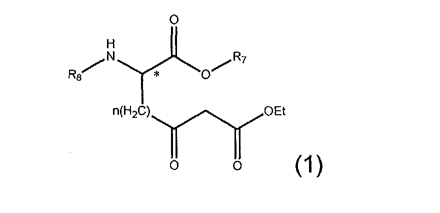 wherein R₇ and R₈ both mean, independently one from each other, a protective group,
with a substituted phenol of the following formula (11) : 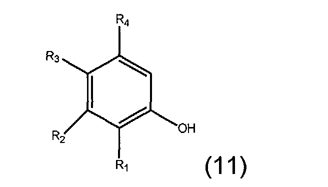 in the presence of methanesulfonic acid, for obtaining the compound of formula (I).

2. The method of claim 1 wherein, for the compound of formula (1), n is an integer ranging from 1 to 2.

3. The method of claim 1 wherein, for the compound of formula (1), n means 1 and step (d) comprises the following steps :
(b1) hydrogenolysis of the compound of formula (1) 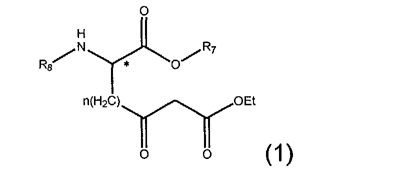 in the presence of catalytic palladium, for obtaining the compound of formula (2) 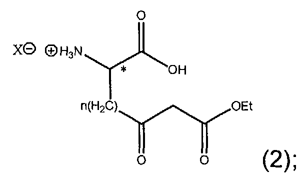 ; and
(b2) reacting the compound of formula (2) with the compound of formula (11) 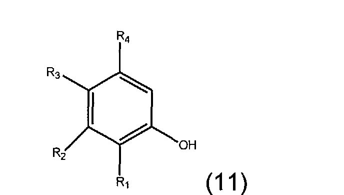 in the presence of methanesulfonic acid, for obtaining the compound of formula (I).

4. The method of any one of claims 1 to 3, wherein the amino acid - ketoester compound of formula (1) is obtained through the steps of :
(a1) subjecting a protected amino acid of the following formula (IV) 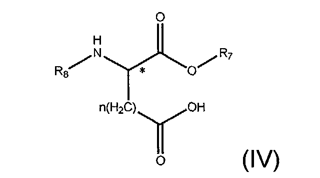 by treatment with carbonyldiimidazole; and
(a2) reacting the activated compound obtained at step (a) with a salt of monoethyl malonic acid, for obtaining wherein the amino acid -ketoester compound of formula (1)

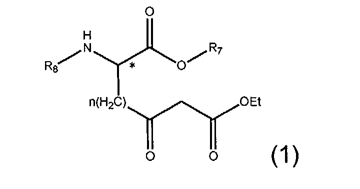

5. A method for manufacturing an optically pure coumaryl amino acid that is protected on its amino group, wherein said method comprises the steps of :
(A) obtaining the compound of formula (I) according to the method of any one of claims 1 to 4; and
(c) reacting the compound of formula (I) obtained at step (A) with the appropriate protective group, whereby obtaining the protected compound of formula (II) : 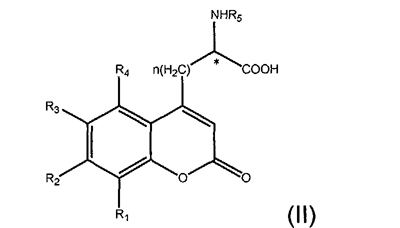
wherein R₅ is a protective group.

6. A method for manufacturing an optically pure coumaryl amino acid that is protected on its carboxyl group, wherein said method comprises the steps of :
(A) obtaining the compound of formula (I) according to the method of any one of claims 1 to 4; and
(d) reacting the compound of formula (I) obtained at step (A) with the appropriate protective group, whereby obtaining the protected compound of formula (III) : 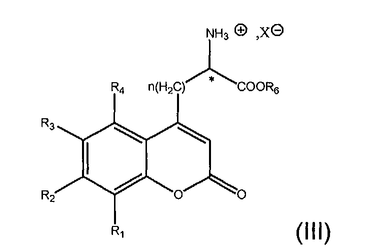
wherein R₆ is a protective group.

7. An optically active coumaryl amino acid salt having the following formula (I) : 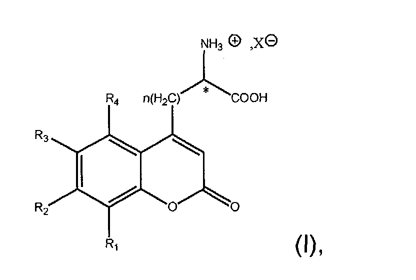 wherein :
(i) n is an integer ranging from 1 to 2 ;
(ii) X⁻ represents Cl⁻, Br⁻, I⁻, CH₃SO₃⁻, CF₃CO₂⁻, CF₃SO₃⁻ ;
(iii) R₁ represents H, halogen, alkyl, acyl, nitrile, sulfonate, aminosulfonyl, carbonyl and carbamoyl, OH, O- or N- substituted by alkyl or acyl group ;
(iv) R₂ represents H, halogen, alkyl, acyl, nitrile, sulfonate, aminosulfonyl, carbonyl and carbamoyl, OH, O- or N- substituted by alkyl or acyl group ; (v) R₃ represents H, halogen, alkyl, acyl, nitrile, sulfonate, aminosulfonyl, carbonyl and carbamoyl, OH, O- or N- substituted by alkyl or acyl group ;
(vi) R₄ represents H, halogen, alkyl, acyl, nitrile, sulfonate, aminosulfonyl, carbonyl and carbamoyl, OH, O- or N- substituted by alkyl or acyl group ; and
(vii) "*" represents the position of an asymmetric carbon atom;

8. The optically active coumaryl amino acid salt according to claim 7, which is selected from the group consisting of :
- (1 S)-1-carboxy-2-(7-hydroxy-2-oxo-2H-chromen-4-yl)-ethyl ammonium trifluoroacetate,
- (1S)-1-carboxy-2-(7-methoxy-2-oxo-2H-chromen-4-yl)-ethyl ammonium methanesulfonate,
- (1 R)-1-carboxy-2-(7-methoxy-2-oxo-2H-chromen-4-yl)-ethyl ammonium trifluoroacetate,
- (1S)-1-carboxy-2-(6-chloro, 7-hydroxy-2-oxo-2H-chromen-4-yl)-ethyl ammonium trifluoroacetate,
- (1S)-1-carboxy-2-(7-ethoxy-2-oxo-2H-chromen-4-yl)-ethyl ammonium trifluoroacetate,
- (1 S)-1-carboxy-2-(5-hydroxy, 7-methoxy-2-oxo-2H-chromen-4-yl)-ethyl ammonium trifluoroacetate,
- (1S)-1-carboxy-2-(7-hydroxy, 5-methoxy-2-oxo-2H-chromen-4-yl)-ethyl ammonium trifluoroacetate,
- (1S)-1-carboxy-2-(5,7-dimethoxy-2-oxo-2H-chromen-4-yl)-ethyl ammonium trifluoroacetate,
- (1S)-1-carboxy-2-(5,7-dihydroxy-2-oxo-2H-chromen-4-yl)-ethyl ammonium trifluoroacetate;
- (1S)-1-ethoxycarbonyl-2-(6,7-dimethoxy-2-oxo-2H-chromen-4-yl)-ethyl ammonium trifluoroacetate; and
- (1S)-1-carboxy-2-(5-hydroxy-7-methoxy-2-oxo-2H-chromen-4-yl)-ethyl ammonium trifluoroacetate.
- (1S)-1-carboxy-3-(7-methoxy-2-oxo-2H-chromen-4-yl)-propyl ammonium trifluoroacetate.

9. A compound of formula (II) according to claim 5 which is selected from the group consisting of :
- (2S)-2-Fmoc-amino-3-(7-methoxy-2-oxo-2H-chromen-4-yl)propionic acid,
- (2S)-2-Cbz-amino-3-(7-methoxy-2-oxo-2H-chromen-4-yl)propionic acid,
- (2S)-2-Boc-amino-3-(7-methoxy-2-oxo-2H-chromen-4-yl)propionic acid; and
- (2R)-2-Fmoc-amino-3-(7-methoxy-2-oxo-2H-chromen-4-yl)propionic acid.

10. A compound of formula (III) according to claim 6 which consists of (1 S)-1-benzyloxycarbonyl-3-(7-methoxy-2-oxo-2H-chromen-4-yl)-propyl ammonium trifluoro-acetate.

11. A kit for manufacturing a fluorescent polypeptide, wherein said kit comprises one coumaryl amino acid derivative selected from the compounds of formula (I), (II) and (III), as defined in claims 1, 5 and 6.

12. A method for the synthesis of an optically active polypeptide wherein said method comprises at least one step of incorporating an optically active coumaryl amino acid of formula (I) within the amino acid chain.

13. An optically active polypeptide which contains in its amino acid chain an optically active coumaryl amino acid of formula (I).

14. An *in vitro* assay kit comprising an optically active polypeptide, which polypeptide contains in its amino acid chain an optically active coumaryl amino acid of formula (I).


SEQUENCE LISTING

<110> Institut National de la Sante et de la Recherche M

<120> A method for manufacturing optically active coumaryl
      amino acids and the novel coumaryl amino acids thus
      obtained

<130> Q895-EP-INSERM

<140>
<141>

<160> 2

<170> PatentIn Ver. 2.1

<210> 1
<211> 17
<212> PRT
<213> Artificial Sequence

<220>
<221> MOD_RES
<222> (17)
<223> Modified Alanine of formula :
      (2S)-2-(Fmoc-amino)-3-(7-methoxy-2-oxo-2H-chromen-
      4-yl)propionic acid

<220>
<223> Description of Artificial Sequence:fluorescent
      peptide

<400> 1
Arg Gln Ile Lys Ile Trp Phe Gln Asn Arg Arg Met Lys Trp Lys Lys
  1               5                  10                  15

Xaa


<210> 2
<211> 17
<212> PRT
<213> Artificial Sequence

<220>
<221> MOD_RES
<222> (17)
<223> Modified Alanine of formula :
      (2S)-2-(Fmoc-amino)-3-(7-methoxy-2-oxo-2H-chromen-
      4-yl)propionic acid

<220>
<223> Description of Artificial Sequence:fluorescent
      peptide

<400> 2
Arg Gln Ile Lys Ile Trp Phe Pro Asn Arg Arg Met Lys Trp Lys Lys
  1               5                  10                  15

Xaa